# EUROPEAN PATENT APPLICATION

(11) **EP 3 757 201 A1**
(43) Date of publication of application: **30.12.2020**
(21) Application number: 19182811.0
(22) Date of filing: 27.06.2019
(51) Int. Cl.: C12M 1/00, C02F 3/10, C12M 1/12

(54) **BIOREACTOR AND METHOD FOR TREATING AT LEAST ONE FLUID AND/OR CULTIVATING PHOTOTROPHIC ORGANISMS**

(71) Applicant: Phytolinc UG, 50937 Köln (DE)
(72) Inventor: Zaabe, Jan-Nicolas, 50939 Köln (DE); Maercker, Arne, 50823 Köln (DE); Prausse, Dennis, 50935 Köln (DE)
(74) Representative: Remus, Alvaro Johannes

(57) **Abstract**

The invention relates to a bioreactor 20 for treating at least one fluid and/or cultivating phototrophic organisms, comprising at least one fluid path and at least one first substrate 29. The first substrate 29 is permeable to fluid but impermeable to phototrophic organisms and comprises at least one first surface 30 for carrying the phototrophic organisms, wherein the first substrate 29 is disposed such that at least the first surface 30 is exposed to ambient air. The first substrate 29 further comprises at least one second surface which is at least partially in contact with the fluid path. According to the invention a circuit for circulating the fluid is provided, wherein the circuit comprises the fluid path and at least one fluid processing unit 28 which is in fluid communication with the fluid path. The invention further concerns an arrangement comprising the bioreactor, wherein the bioreactor is in fluid communication with at least one aquaculture system and waste water derived from the aquaculture system is processed in the bioreactor and then recirculated to the aquaculture system. The invention also relates to a method for treating at least one fluid and/or cultivating phototrophic organisms, wherein the fluid is processed by means of the fluid processing unit.

## Description

### Background of the invention

The invention relates to a bioreactor for treating at least one fluid and/or cultivating phototrophic organisms, comprising at least one fluid path and at least one first substrate, said first substrate being permeable to fluid but impermeable to phototrophic organisms and comprising at least one first surface for carrying the phototrophic organisms, wherein the first substrate is disposed such that at least the first surface is exposed to ambient air, and wherein the first substrate further comprises at least one second surface which is at least partially in contact with the fluid path. The invention further concerns a method for treating at least one fluid and/or cultivating phototrophic organisms, wherein phototrophic organisms are immobilized on at least one first surface of at least one first substrate, wherein said first surface is exposed to ambient air, and wherein said first substrate is permeable to fluid but impermeable to phototrophic organisms and further comprises at least one second surface which is at least partially in contact with a fluid path which is in fluid communication with at least one fluid processing unit.

### Prior art

Living phototrophic organisms such as microalgae and cyanobacteria are an essential feed component for the production of fish, crustaceans, and muscles. Moreover, microalgae have a high level of protein and vitamin B12 and contain valuable omega fatty acids as well as natural pigments. Therefore, they serve as raw material in the food supplement and cosmetics industry and are cultivated by microalgae producers. For economic reasons it is advantageous to use waste or process water from other processes as nutrient source. Possible fields of application to recycle nutrients created during the production are aquaculture facilities. These facilities produce aquatic organisms under controlled conditions. Nutrients accumulate in the water through the metabolic products of animals as well as feed residue. As a consequence, the water quality decreases, which limits the productivity. To solve this problem, closed recirculating systems were invented. In these systems, the water constantly passes biological and mechanical filtration processes and is afterwards fed back into the animal pond. This process accumulates nitrogen in the facility which must be constantly washed out through the addition of fresh water. Nitrogen compounds however serve as excellent nutrient source for plants. Therefore, aquaponics systems, which combine the cultivation of fish and plants, have emerged.

Since many commercially relevant plants naturally do not live in aquatic conditions, additional adjustments of the waste or process water are necessary. These might be adjustments to pH, temperature, or salt concentration. However, phototrophic organisms such as microalgae and cyanobacteria naturally live in aquatic environments and can perfectly be adjusted according to the other processes. Commercial producers cultivate phototrophic organisms like microalgae and cyanobacteria in open ponds, tubular or plate-shaped photobioreactors. The underlying cultivation technique for all commercially available photobioreactors is suspension culture in which the algae cells are cultivated free-swimming in a water column. High costs for the concentration of algae biomass from the medium suspension as well as costly mechanical harvesting and the following drying of the algae cells are drawbacks of suspension culture based systems. Also, the increasing cell density makes an optimal lighting of the cells inside the suspension culture more difficult.

Production facilities for phototrophic organisms in suspension culture can be combined with a nutrient source from another process, e.g. the production of aquatic organisms, in a complex aquaponics system. Such state of the art production facilities may include a batch process which offers a possible production mode for the cultivation of phototrophic organisms in photobioreactors. Thereby, a predefined volume of nutrient solution is fed into the photobioreactor until a certain algae cell density is reached. Then, the algae cells are separated from the suspension in a mechanical process under energy input. If the nutrient concentration is high, the point of harvest can be determined so that the algae cells are harvested at an optimal cell density. If, after harvesting the algae cells, the nutrient concentration is still above the desired level, further nutrient solution is fed into the reactor and the cultivation step is repeated. This loop is repeated until the desired nutrient level is reached or until (almost) all nutrients are removed from the water. During each repetition, the algae cells must be separated from the suspension in a complex and costly process.

If the nutrient concentration of the water, which has been initially fed into the reactor, is too low to reach the optimal harvesting point, the algae cells are separated from water as soon as all nutrients are used up. Subsequently, the reactor is re-filled with nutrient solution, which has previously passed through the process of water treatment. Now, the previously harvested algae biomass is used to inoculate the photobioreactor. Since the algae biomass is harvested during a period of increasing growth rates, this procedure increases the overall algae biomass productivity. This procedure is repeated until the cell density reaches a level at which growth rates decrease due to high shading. The additional extraction steps though are inefficient and expensive.

A further production method represents the continuous mode. Thereby, nutrient solution flows continuously into the reactor. Simultaneously algae suspension flows out of the reactor, such that the volume of outflowing algae suspension culture equals the volume of inflowing nutrient solution. If size and density of the suspension culture are not optimally adjusted to the nutrient concentration of the medium, unused nutrients flow back into the water cycle. As in the batch mode, the algae cells are separated from the suspension culture after the cultivation through the use of energy and either harvested in form of concentrate or refed into the reactor. The necessary separation processes as well as the circulation of masses of water are energy-intensive and costly.

As known from the state of the art today, the productivity of a bioreactor (based on suspension culture) depends on the biomass concentration inside the reactor. Furthermore, mechanical extraction methods for the separation of algae cells and suspension culture are a known stress factor for the algae. As a consequence, productivity decreases. However, for the process method of interest (production of phototrophic organisms, while waste or process water from other processes, such as the production of aquatic organisms, serves as nutrient source) mechanical extraction processes are necessary in order to optimize the biomass concentration inside the reactor.

It is known that phototrophic organisms such as microalgae and cyanobacteria are suitable for water purification since they absorb and bind various nutrients. For example, DE 10 2014 018 697 A1 discloses a production facility for cultivating phototrophic organisms such as microalgae and cyanobacteria using waste or process water from other processes as nutrient source. The facility comprises a reactor circuit with a reactor pump, a supply circuit having a supply pump for processing the waste or process water and supplying the reactor circuit with the processed water, and a dialysis membrane separating the supply circuit from the reactor circuit but allowing a diffusive mass transfer limited by the pore size of the dialysis membrane.

Such state of the art suspension cultivation systems, in which the algae cells grow free-swimming in a water column, require a large volume of water. It is known that ensuring hygienic conditions inside the reactor requires a lot of effort. The limiting factors are the large water volumes which pass different purification and treatment steps. This is a very energy intensive and costly process but necessary to maintain the desired water quality. As stated above, these processes face severe drawbacks concerning the production of phototrophic organisms, when cultivated with waste or process water from other processes as a nutrient source.

US 6 013 511 A discloses a reactor containing microorganisms immobilized on a membrane made of an inorganic oxide and an organic polymer such as polysulfone. The membrane has a skin side and an open side, wherein the pores of the skin side are smaller than pores of the open side. The microorganisms are immobilized as a biofilm on the skin side of the membrane. A nutrient chamber supplies a nutrient solution to the open side of the membrane and the nutrient solution passes through the membrane from the open side to contact the microorganisms on the skin side. An effluent chamber supplies an effluent solution containing a metal in the form of a salt, a xenobiotic compound such as a chlorinated organic compound or both to the biofilm of microorganisms on the skin side, and the microorganisms precipitate the metal and/or degrade the xenobiotic compound. The concentration of nutrients in the nutrient chamber is kept sufficiently low so that the microorganisms of the biofilm utilize essentially all nutrients that pass through the membrane to prevent essentially any nutrients from entering the effluent solution. The membrane separates the effluent chamber and nutrient chamber such that leakage between the chambers is prevented.

Membrane based photobioreactors reduce the water consumption for algae biomass production while also the gas exchange is improved (Podola, Li, and Melkonian: "Porous Substrate Bioreactors: A Paradigm Shift in Microalgal Biotechnology?", Trends in Biotechnology, February 2017, Vol. 35, No. 2, 121-132). For example, WO 2005/010140 A1 discloses a method and device for cultivating eukaryotic microorganisms, whereby a perforated support having a first main surface and a second main surface, which is substantially impermeable to eukaryotic microorganisms, is prepared and the microorganisms are applied on the first main surface. A film containing an aqueous solution passes over the second main surface. Said aqueous solution moves from the second main surface to the first main surface substantially by means of capillary forces. As a result, the first main surface is supplied with aqueous solution and the applied microorganisms grow on said first main surface.

WO 97/11154 A1 discloses a photobioreactor comprising a revolving membrane and a spraying system to supply the algae surface with nutrient solution. Thereby, biomass productivity increases and water consumption decreases. However, during the spraying process the algae cells detach from the membrane and suspend in the medium culture.

The problems of current production processes of phototrophic organisms which are cultivated with waste or process water from other processes as nutrient source (e.g. production of aquatic organisms) concern the inefficient use of available nutrients in the water. State of the art technology solves these problems by controlling the cell density in the reactors through mechanical extraction. But currently applied processes to increase the cell density in the reactor are energy intensive and stress the produced organisms. Additionally, the inefficient use of available nutrients leads to a high water consumption. Since water treatment is an essential but also costly process, this inefficiency can make the production unprofitable.

### Summary of the invention

It is an object of the invention to solve the above-mentioned problems of prior art systems and methods and to provide a bioreactor and method for treating at least one fluid and/or cultivating phototrophic organisms with increased productivity per square meter of surface area.

It is another object of the invention to provide a bioreactor and method for treating at least one fluid and/or cultivating phototrophic organisms with which the risk of contamination of the fluid can be reduced and effectivity of water treating can be enhanced.

The object is met by a bioreactor as initially specified, wherein a circuit for circulating the fluid is provided, said circuit comprising the fluid path and at least one fluid processing unit which is in fluid communication with the fluid path. By the provision of a (closed) fluid circuit within which the fluid can circulate through a fluid processing unit and the fluid path along the second surface of the first substrate, the amount of fluid (e.g., waste water or culture medium) necessary for cultivation of the phototrophic organisms can be significantly reduced. Integrating a fluid processing unit in the fluid circuit allows for efficient fluid control and treatment in immediate vicinity of the fluid path and the second surface of the first substrate. Controlling and processing (i.e., purifying and/or sterilizing) the fluid immediately before it is provided to the phototrophic organisms is particularly beneficial because, due to the short distance between the fluid processing unit and the fluid path, the risk of contamination is minimized and an interim change of the fluid's quality can be excluded. Moreover, due to the reduced amount of fluid, effectivity of fluid processing (e.g., sterilization and/or purification) is enhanced as the volume to be treated and the flow rate can be minimized. Using a (closed) circuit further simplifies monitoring and maintenance of the fluid's quality and thus decreases the risk of contamination of the phototrophic organisms and minimizes the effort for adjusting the fluid's characteristics. That is, control of the entire fluid treatment and cultivation process is simplified. It is another advantage of the bioreactor according to the invention that the efficiency of the use of available nutrients in the circulating water volume is increased. The almost complete utilization of the nutrients leads to increased productivity per square meter of surface area so that the efficiency of the entire cultivation process is significantly enhanced. Further, as the quality of a fluid circulating in a (closed) circuit can be monitored and maintained easily, additional costly fluid treatment steps and/or as waste of fluid can be avoided.

According to an advantageous embodiment of the invention the fluid processing unit comprises at least one sterilizing device. Keeping the fluid within the fluid circuit as sterile as possible is essential for cultivation of phototrophic organisms. Especially if waste or process water from other processes is used as nutrient source, the water has to be purified and then sterilized thoroughly. This is necessary due to different organisms in the waste or process water which may contaminate the culture of phototrophic organisms and thus can make their cultivation/expansion impossible. It is therefore beneficial if a sterilizing device, for example, at least one ultraviolet (UV) light source (UV clearer) and/or at least one ozone generator, is integrated in the fluid processing unit. Sterilizing the fluid immediately before it is provided to the phototrophic organisms is of particular importance since by this measure, and especially due to the short distance between the fluid processing unit and the fluid path, the risk of contamination of the culture can be significantly reduced. In another advantageous embodiment of the invention the fluid processing unit further comprises at least one device selected from the group consisting of a filter element, a cooling and/or heating device, ventilation equipment, a heat exchanging device (recuperator), a pressure reducer, a pump, a pH meter, a conductivity meter, a thermometer, and a manometer. Including all devices that are necessary to provide optimal conditions for cultivation in the fluid processing unit ensures perfect preparation of the fluid in immediate vicinity of the fluid path and thus the phototrophic organisms. The additional devices enable the fluid processing unit to control the fluid characteristics and to adjust parameters that differ from optimal cultivation conditions. Controlling and processing the fluid, in particular purification and sterilization of the fluid and adjustment of its temperature, pH value or conductivity, is of crucial importance for culturing/producing the phototrophic organisms. For example, in order to cultivate phototrophic organisms such as microalgae and cyanobacteria with nutrients from process or waste water, monitoring of the fluid's quality and complex purification processes are absolutely necessary in order to keep the algae culture clean.

In a further advantageous embodiment of the invention at least one second substrate is provided, said second substrate comprising at least one first surface for carrying phototrophic organisms, wherein the first substrate is disposed such that at least the first surface is exposed to ambient air, and at least one second surface which is at least partially in contact with the fluid path. Providing two or more substrates allows for easy expansion of the area available for production of the phototrophic organisms. In a special embodiment the first substrate and the second substrate may be arranged such that their respective second surfaces delimit the fluid path. That is, two flat, sheet- or plate-like substrates can be arranged parallel to each other so as to build a kind of channel representing the fluid path. In this advantageous sandwich-like configuration the fluid can flow between the two substrates without the need to provide additional elements for guiding the fluid flow. Upscaling of the bioreactor's capacity can be easily achieved by multiplying the number of substrates.

In another special embodiment a substrate itself (i.e. the first substrate and/or any further substrate) can have a sandwich-like configuration wherein a second surface and a third surface of this substrate are arranged such that they delimit a fluid path so that the fluid path is part of the substrate.

In order to ensure even and complete wetting of the second surface of the substrate, the fluid path may comprise at least one porous material being suitable to evenly spread the fluid within the fluid path. Preferably, the porous material may comprise a spacer fabric or the like. For example, the porous material, such as a spacer fabric or the like, may be part of a substrate and can be disposed in the fluid path between two sheets or membranes, i.e. the second and the third surface.

According to an advantageous embodiment of the invention the first substrate and/or the second substrate comprise(s) a microporous membrane comprising polyethersulfone (PES). Such membranes are permeable to fluid but impermeable to phototrophic organisms and well-suited for carrying organisms like algae and cyanobacteria. As described in US 6 013 511 A, such organisms can be easily immobilized on PES membranes.

In order to further enhance the affinity of phototrophic organisms to specific substrates (e.g., PES membranes), the first surface may comprise an immobilizing material for bearing at least one biofilm comprising the phototrophic organisms. The immobilizing material can be a compound which modifies the substrate's surface or a substance which serves as a link between the surface and the organisms.

According to a further advantageous embodiment of the invention the bioreactor may additionally comprise a control unit comprising at least one sensor device for monitoring and regulating the processes employed by the bioreactor. Such sensor device can be designed, for example, to measure at least one condition selected from the group consisting of temperature, CO₂ concentration, light intensity [µmol/s/m²], filling level, and humidity. Measuring and monitoring such parameters is an essential requirement for ensuring optimal conditions for culturing/producing the phototrophic organisms.

In a further advantageous embodiment of the invention a container for collecting the fluid is provided, said container being in fluid communication with the fluid path and the fluid processing unit, wherein at least one connecting element comprising a beveled edge is disposed between the fluid path and the container. Excess fluid from the fluid path and fluid that is not evaporated or has not been absorbed by the substrate or the phototrophic organisms is collected in a container and then fed into the fluid processing unit for processing before it is recirculated into the fluid path. In order to prepare the fluid for processing, it can be stirred in the container by means of a suitable mixing device such as a circulation pump, so as to equalize possible concentration differences in the fluid. Preferably, a connecting element comprising a beveled edge is disposed between the fluid path and the container, so that the fluid can flow/drop directly into the container without splashing. Preventing splashing of the fluid upon entering the container is important in order to avoid that splashes reach the first surface of the substrate and thus the phototrophic organisms (biofilm). The container may comprise at least one sensor device for measuring and monitoring at least one specific parameter in the fluid. Such sensor device can be designed, for example, to measure at least one parameter selected from the group consisting of temperature, CO₂ concentration, opacity, and filling level.

The invention also concerns an arrangement comprising the bioreactor as described herein, wherein the bioreactor is in fluid communication with at least one aquaculture system, and wherein waste water derived from the aquaculture system is processed in the bioreactor and then recirculated to the aquaculture system. In such aquaponics systems the nutrients for the phototrophic organisms such as algae and cyanobacteria are provided by waste or process water from other processes, such as the production of aquatic organisms. The use of the bioreactor according to the invention in aquaponics systems or the like is therefore a further advantageous aspect of the invention.

The object is further met by a method as initially specified, such method comprising:
a) Processing the fluid by means of the fluid processing unit,
b) providing the fluid by the fluid processing unit to the fluid path,
c) bringing at least a part of the fluid in contact with the second surface of the first substrate, wherein the first surface is provided with the fluid through the second surface,
d) recirculating the remaining fluid from the fluid path to the fluid processing unit, and
e) repeating steps a) to d) at least once.

That is, according to the invention the fluid flows through the fluid processing unit and the fluid path along the second surface of the first substrate so that the first surface and thus the phototrophic organisms are provided with the fluid before the remaining fluid (i.e. excess fluid from the fluid path and fluid that is not evaporated or has not been absorbed by the substrate or the phototrophic organisms) is recirculated to the fluid processing unit. Two or more cycles of processing and providing of the fluid to the organisms can be performed until the treating/cultivation process is completed. By this circulation of the fluid (e.g., waste water or culture medium) the amount of fluid necessary for cultivation of the phototrophic organisms can be significantly reduced. As the processing of the fluid is integrated in the circuit efficient fluid control and treatment in immediate vicinity of the fluid path is possible. Controlling and processing (i.e., purifying and/or sterilizing) the fluid immediately before it is provided to the phototrophic organisms is particularly beneficial because the risk of contamination is minimized and an interim change of the fluid's quality can be excluded. Moreover, due to the reduced amount of fluid, effectivity of fluid processing (e.g., sterilization and/or purification) is enhanced as the volume to be treated and the flow rate can be minimized. Letting the fluid circulate within a reactor device simplifies monitoring and maintenance of the fluid's quality and thus decreases the risk of contamination of the phototrophic organisms and minimizes the effort for adjusting the fluid's characteristics. That is, control of the entire fluid treatment and cultivation process is simplified. It is another advantage of the method according to the invention that the efficiency of the use of available nutrients in the circulating water volume is increased. The almost complete utilization of the nutrients leads to increased productivity so that the efficiency of the entire cultivation process is significantly enhanced. Further, as the quality of a circulating fluid can be monitored and maintained easily, additional costly fluid treatment steps and/or as waste of fluid can be avoided.

In an advantageous embodiment of the method according to the invention the processing of the fluid comprises at least one sterilization process. Sterilizing the fluid immediately before it is provided to the phototrophic organisms is highly beneficial because by this measure the risk of contamination of the culture can be significantly reduced. Keeping the circulating fluid as sterile as possible is essential for cultivation of phototrophic organisms. For example, the sterilization process may comprise at least one UV irradiation and/or at least one ozone treatment of the fluid.

In another advantageous embodiment of the method according to the invention the processing of the fluid comprises at least one treatment selected from the group consisting of filtration, cooling, heating, pH adjustment, addition of water and/or medium, and adjustment of gas content. Preparing the fluid for the cultivation of phototrophic organisms by processing it for the purpose of, for example, purification and sterilization, adjustment of temperature, pH value or conductivity, is necessary for ensuring the provision of optimal conditions for culturing/producing the phototrophic organisms.

The fluid can be a liquid solution comprising nutrient compounds for cultivating phototrophic organisms. For example, the fluid may be a culture medium adapted to the needs of phototrophic organisms or any other kind of defined liquid that is suitable for cultivating phototrophic organisms. Additionally or alternatively, the fluid can be waste water derived from an aquaculture, wherein at least some of the waste is removed from the water by the phototrophic organisms and the cleaned water is then recirculated to the aquaculture. That is, the invention relates to a production facility for phototrophic organisms in which waste and process water of other processes (e.g. the production of aquatic organisms) is used as nutrient source.

The phototrophic organisms cultivated/produced by the method according to the invention can be removed from the substrate and then directly fed to an aquaculture. Alternatively, the phototrophic organisms can be removed from the substrate and then used to produce fatty acids, protein, food and/or dietary supplements.

### Brief description of the figures

**Figure 1** shows a schematic overview representation of a facility for production of phototrophic organisms including a bioreactor according to the invention.
**Figure 2** shows a perspective representation of an embodiment of a bioreactor according to the invention.
**Figure 3** shows a perspective representation of the bioreactor according to Figure 2 without substrate and lighting devices.
**Figure 4** shows a different perspective of the bioreactor according to Figure 3.
**Figure 5** shows a detailed perspective representation of the cover plate of the container of the bioreactor according to Figure 2.
**Figure 6** shows a front view inside an embodiment of the fluid processing unit of the bioreactor according to Figure 2.

### Detailed description of exemplary embodiments of the invention

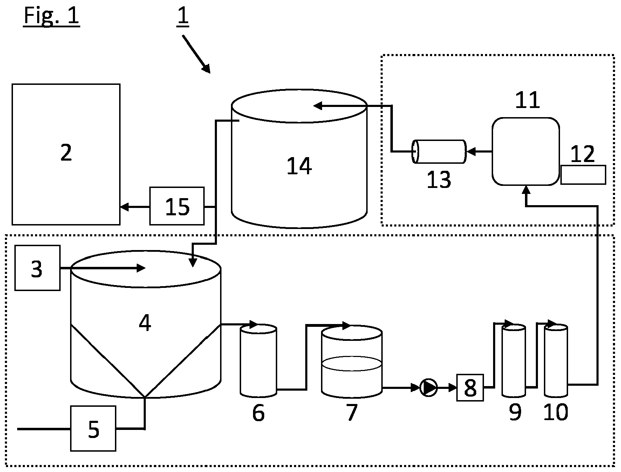

**Figure 1** shows an exemplary facility 1 for production of phototrophic organisms including a bioreactor 2 according to the invention. With the facility 1 treatment of waste or process water from other processes such as the production of aquatic organisms is accomplished. The facility 1 comprises a water feed 3 through which the water volume is supplied with nutrients provided through waste or process water from other processes. The water flows through one or more sedimentation tank(s) 4. After flooding the sedimentation tank 4, the water remains in there for a period of one to five hours. Thereby, coarse dirt particles sediment on the tank bottom. By opening a valve 5 at the bottom of the conically shaped tank 4, the sediment particles are washed out of the system. After having been pre-filtered by the sedimentation process, the water can be pumped into a disc and/or sieve filter 6 with a pore size of 120 - 130 µm. After having been pre-filtered by the disc and/or sieve filter 6, the water can then be pumped through a sand filter 7. There, the filtration with a pore size of 80 - 20 µm takes place. Thereby, larger organic or inorganic molecules are removed from the water. After having been filtered by the sand filter 7, the purified water with particles of 20 - 80 µm is pumped through a pressure reducer 8 into one or more filter element(s) 9 for depth filtration with a pore size of 10 - 50 µm. Then, the water is pumped in one or more filter element(s) 10 for the depth filtration with a pore size of 0,5 - 5 µm. The filtration steps as described above lead to a nutrient solution nearly free of suspended matter. After the filtration process A, the sterilization process B takes place. Thereby the water can be passed through one or more oxidation system(s) 11 as well a compressor 12. The compressor 12 feeds the produced ozone into the oxidation system 11, in which an oxidative flotation sterilizes the pre-filtered water. Moreover, the pre-treated water can be passed through one or more UV clarifier(s) 13. To ensure a sufficient sterilization of the waste or process water the Watt power must not be set too low. The purified water, which originates from process or waste water of other processes and shall serve as nutrient source for the production of phototrophic organisms, is then fed into the production unit of the phototrophic organisms. At first it is collected in one or more collection tank(s) 14. The nutrient solution is fed from collection tank 14 through a valve into one or more bioreactor(s) 2 (e.g., at least one photobioreactor). The nutrient medium is caught in the tank of the bioreactor 2 until the desired filling level is reached. If no further nutrient medium is needed, the pre-treated nutrient solution flows through one or more overflows back into the one or more sedimentation tank(s) 4. There, the filtration process (A) and the sterilization process (B) start again. This ensures a sufficient amount of pre-treated nutrient solution at all times.

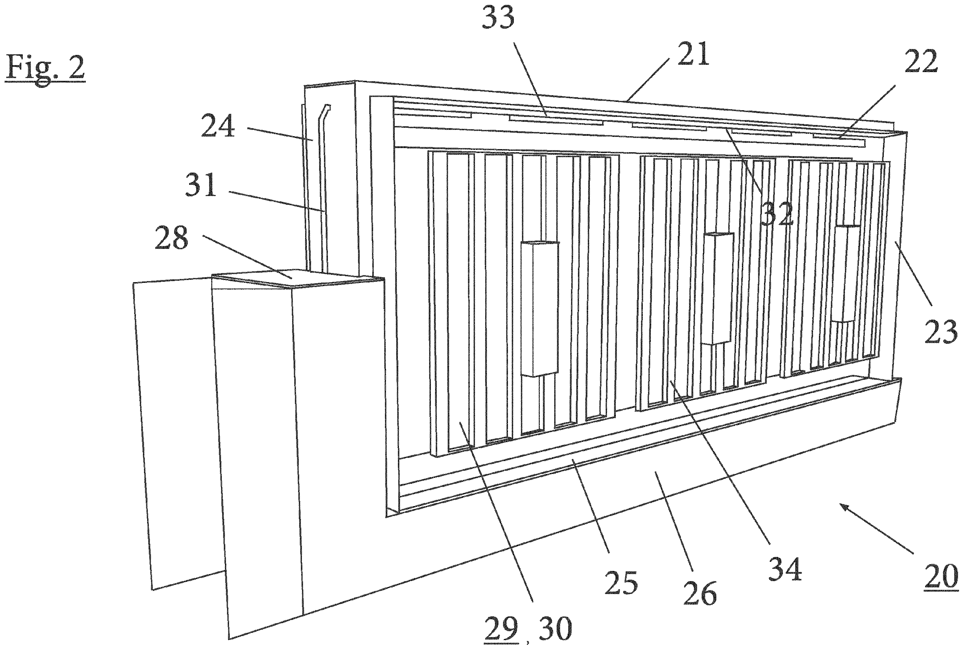

**Figure 2** shows an embodiment of a bioreactor 20 according to the invention. The bioreactor 20 is a photobioreactor with an exemplary size of 4000 x 2000 x 400 mm. The component parts of the bioreactor 20 are preferably at least in part made of food-safe polyethylene (PE). The bioreactor 20 comprises at its top a cross brace 21 and a first substrate suspension 22, the first substrate suspension 22 being disposed in parallel to the cross brace 21 and attached to it. A second substrate suspension (not visible) is attached to the cross brace 21, wherein this second substrate suspension is disposed in parallel to the first substrate suspension 22. The bioreactor 20 further comprises two side supports 23, 24 which connect the cross brace 21 to a cover plate 25 at the bottom of the bioreactor 20. A container 26 (e.g., a plastic tank) is welded or glued to the cover plate 25 (e.g., a plastic plate). Two connecting elements (not visible) forming a drainage channel (not visible) are glued or welded to the cover plate 25 at the top of the container 26. Furthermore, the bioreactor 20 comprises a small fluid processing unit 28 which may include, for example, a membrane pump, a pressure reducer, a manometer, a filter candle with a pore size of 1 µm, and a UV clarifier. Additionally, control sensors, which may include detectors for measuring CO2, temperature, light intensity (µmol/s/m2), pH, conductivity, filling level, leakage, and pressure, as well as other control devices such as pump control (on/off), fan control (on/off/control), LED control (on/off/control), and image recognition of the cultivation surfaces, can be installed in the bioreactor 20.

A first substrate 29 is attached to the substrate suspension 22, at the opposite end to a first connecting element at the drainage channel, and laterally to the side supports 23, 24. The substrate 29 is designed to carry phototrophic organisms (e.g., algae) at a first surface 30 (outer surface) which is exposed to the ambient air. In this embodiment the substrate 29 comprises a microporous membrane having a first surface 30 which is at least partially exposed to ambient air and, at the opposite side of the substrate 29, a second surface (not visible) which is in contact with a fluid (e.g., water, culture medium or another nutrient solution), wherein the membrane is permeable to the fluid so that the fluid can pass from the second surface through the membrane to the first surface. Accordingly, phototrophic organisms such as algae that are immobilized on the first surface 30 can utilize the fluid. In another advantageous embodiment of the invention two substrates (membranes) can be arranged parallel to each other so as to build a channel between them, which represents a fluid path where the fluid can flow between the two substrates. To this end, a second substrate (not visible) is attached to the second substrate suspension, at the opposite end to a second connecting element at the drainage channel, and laterally to the side supports 23, 24. A porous material can be provided in the fluid path in order to ensure even distribution of the fluid. Thus, such sandwich-like configuration comprises three layers, a porous material (e.g., a spacer fabric material) in the middle and two fine-pored membranes (e.g., made of polyethersulfone with pore sizes of 0.2 - 20 µm) on the outer faces. The three layers can be attached to each other by a thermoplastic adhesive.

A pump (e.g., a membrane pump) pumps the fluid from the container 26 through the fluid processing unit 28 and tubing 31 into a fluid distribution bar 32. The fluid distribution bar 32 comprises water droppers 33 attached to it and distributes the fluid evenly over the second surface of the substrate 29 or a porous material between two substrates. The fluid spreads over the second surface or the middle layer (porous material; e.g. a spacer fabric material) and thus provides the first surface(s) 30 of the substrate(s) (fine pored membranes) with the fluid. The phototrophic organisms (e.g., algae cells) are applied to the first surface 30 and immobilized there. The fluid (e.g., a nutrient solution) passes through the pores of the fine pored membrane to the phototrophic organisms (e.g., a biofilm consisting of algae cells) and supplies them with water and nutrients. The bioreactor 20 is further equipped with artificial light sources 34 (LEDs, metal halide lamps) but can also or alternatively be operated with sunlight. Until harvest, the phototrophic organism cells grow on the fine pored membrane and can then be harvested with mechanical forces such as scraping or ultrasound. A chemical treatment with surfactant agents and/or organic solvents is also possible. Further harvesting procedures are possible:
A) The algae biomass is harvested together with the fine pored membrane;
B) Detached biomass from the membrane is collected in flowing medium;
C) Detached and dried biomass from the porous carrier material is collected.
After harvesting, the wet biomass can directly be used as feed for aquatic organisms. Moreover, the biomass can be dried and afterwards be processed as powder or pellets. The powder can be mixed with fish feed. Also, the resulting powder as well as the pellets can be used in the food supplement sector. Products with a high concentration of omega3/6 fatty acids, vitamin B12, or protein are conceivable. If the produced biomass serves as source for pigments, further steps to extract and/or isolate the corresponding pigment are necessary. Usage for pigments from microalgae can be found in the cosmetics or feed industry.

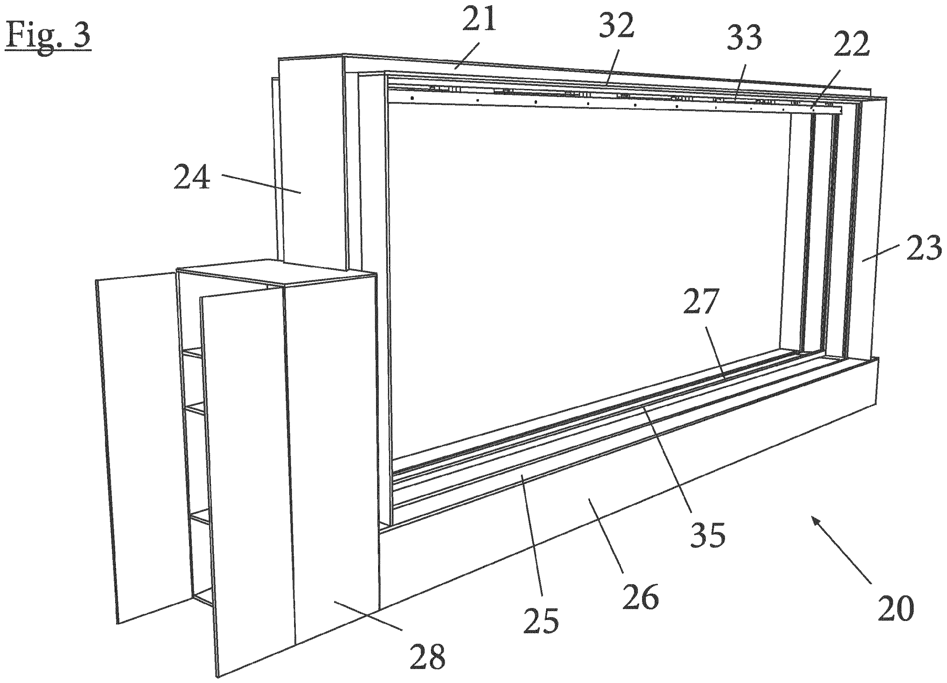

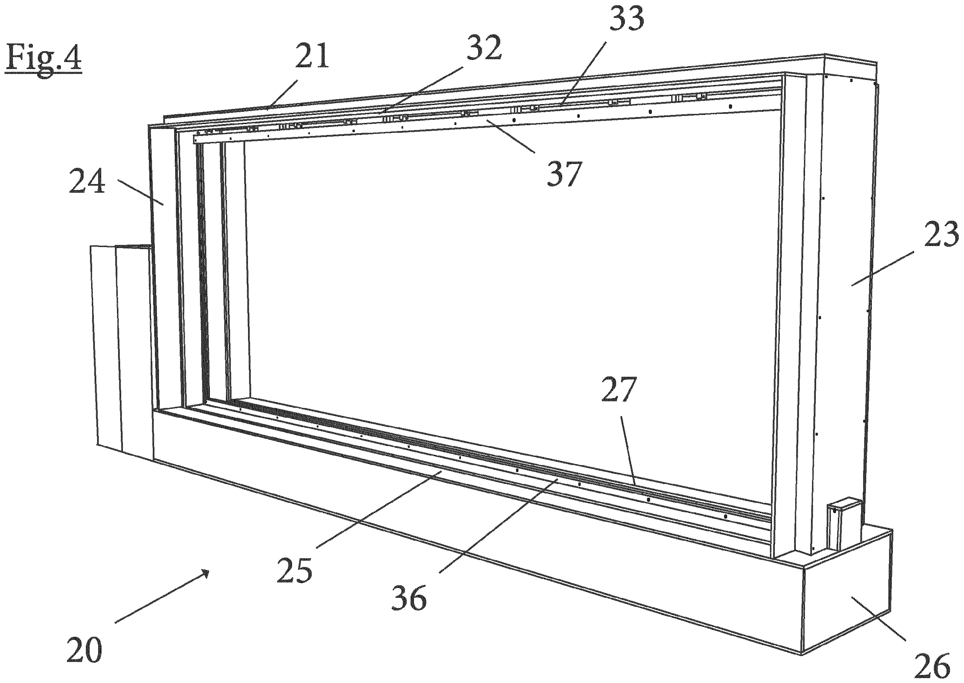

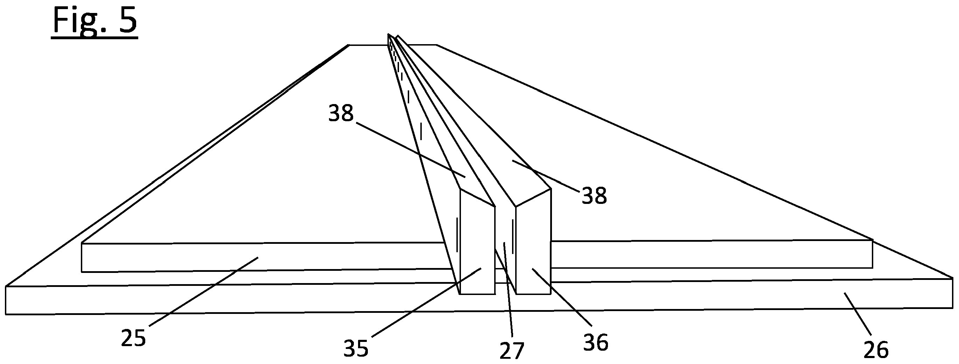

**Figures 3** **and** **4** show an embodiment of the bioreactor 20 according to Figure 2 without substrate and lighting devices. Accordingly, the means for mounting the substrate(s) are visible in this representation. At the top of the bioreactor 20, a first substrate can be attached to the first substrate suspension 22. Laterally, the first substrate can be fixed at the side supports 23, 24. At the bottom of the bioreactor 20, the first substrate can be attached to the first connecting element 35 at the drainage channel 27. The drainage channel 27, which receives the fluid from the fluid path and channels it through an elongated opening of the cover plate 25 into the container 26, is formed by two connecting elements 35, 36. As shown in detail in **Figure 5****,** the two connecting elements 35, 36, which are arranged in parallel to each other, are glued or welded to the cover plate 25 at the top of the container 26. A second substrate can be attached to a second substrate suspension 37, laterally at the side supports 23, 24, and at the bottom of the bioreactor 20 to the second connecting element 36. The first and the second substrate delimit a fluid path where the fluid can flow between the two substrates. Accordingly, the fluid can flow from the fluid distribution bar 32 through the fluid path along the second surfaces of the substrates and the drainage channel 27 into the container 26 where it is collected until it is recirculated into the fluid processing unit 28. The connecting elements 35, 36 each comprise a beveled edge 38 which is disposed between the fluid path and the container 26. Hereby, the fluid can flow/drop directly into the container 26 without splashing so that splashes or fluid drops cannot contaminate the first surface of the substrate and thus the phototrophic organisms (biofilm).

In an advantageous embodiment the side supports 23, 24 of the bioreactor 20 comprise a kind of ventilation shaft within the bioreactor 20 and can be made of a thin-walled sheet metal or plastic so that, due to the fact that the ambient air is cooler that the air within the bioreactor, air moisture condensates at their inner surfaces. The condensate can then flow from the ventilation shaft through the drainage channel 27 into the container 26. That is, the side supports 23, 24 can serve as a kind of cooling trap for dehumidifying the air within the bioreactor, so as to reduce loss of water/fluid during the cultivation process.

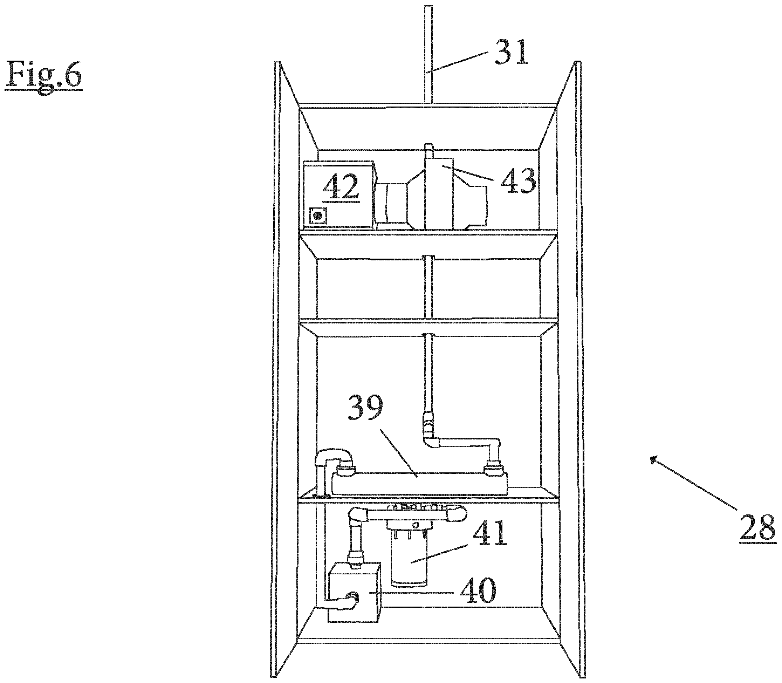

**Figure 6** shows an exemplary embodiment of the fluid processing unit 28 of the bioreactor 20 according to Figure 2. The fluid processing unit 28 is a compact cabinet which is disposed in immediate vicinity of the substrate(s) for cultivating the phototrophic organisms. In particular, the fluid processing unit can be directly mounted to a side support of the bioreactor 20 so that the distance between the equipment of the fluid processing unit 28 and the fluid path is very short. Processing the fluid, i.e. preparing the fluid for the cultivation process, immediately before it is provided to the phototrophic organisms is very advantageous since, by this measure, control of the relevant process parameters is very precise and the risk of contamination of the fluid can be significantly reduced. To this end, the length of the tubing 31, which connects the fluid processing unit 28 to the fluid distribution bar 32, should be as short as possible. The fluid processing unit 28 comprises at least one sterilizing device 39 in order to ensure continuous sterilization and to keep the fluid within the fluid circuit as sterile as possible. This is important because microorganisms in waste/process water or medium which may contaminate the culture of phototrophic organisms could render their cultivation impossible or at least ineffective. In this particular embodiment, the sterilizing device 39 is a UV clearer including an ultraviolet (UV) light source. However, alternatively or additionally, any other sterilizing device which can be integrated in the fluid processing unit 28 may also be suitable. The fluid processing unit 28 may further comprise filter element(s), cooling and/or heating device(s), ventilation equipment, heat exchanging device(s), pressure reducer(s), at least one pump, and devices for measuring the quality of the fluid, such as pH meter, conductivity meter, thermometer, and manometer. Moreover, The fluid processing unit 28 may additionally comprise control devices for controlling the adjustment of the fluid's quality and other process parameters. In this particular embodiment, the sterilizing device 39 further comprises a membrane pump 40, a filter element 41, a heat exchanging device 42, and a ventilator 43.

The combined use of nutrients derived from other processes in the form of waste or process water through the described water treatment and cultivation method as well as the described bioreactor construction provides an innovative process for the production of phototrophic organisms. The use of nutrients, which result from process or waste water of other processes, enables one to leverage economic and ecological synergies. The described water treatment process is necessary to remove various organisms from the process or waste water, which would otherwise contaminate the culture and make a production impossible. This process is energy-intensive and costly. Therefore, it is essential to use nutrients from waste or process water of other processes as efficiently as possible. In all currently known procedures, the concentration of the limited nutrients of the nutrient solution component determine the volumetric productivity of photobioreactors. The above-described bioreactor according to the invention enables the production of algae or other phototrophic organisms with a minimum consumption of water and efficiently separates the biomass from the nutrient solution. Therefore, in the described process the biomass density is independent from the concentration of the limiting nutrient component. Hence, for the first time, an optimal use of nutrients from process or waste water of other processes is possible at a very low cost with very little water treatment effort.

## Claims

1. Bioreactor (20) for treating at least one fluid and/or cultivating phototrophic organisms, comprising at least one fluid path and at least one first substrate (29), said first substrate (29) being permeable to fluid but impermeable to phototrophic organisms and comprising at least one first surface (30) for carrying the phototrophic organisms, wherein the first substrate (29) is disposed such that at least the first surface (30) is exposed to ambient air, and wherein the first substrate (29) further comprises at least one second surface which is at least partially in contact with the fluid path, **characterized in that** a circuit for circulating the fluid is provided, said circuit comprising the fluid path and at least one fluid processing unit (28) which is in fluid communication with the fluid path.

2. Bioreactor according to claim 1, **characterized in that** the fluid processing unit (28) comprises at least one sterilizing device (39), preferably at least one UV light source and/or at least one ozone generator.

3. Bioreactor according to claim 1 or 2, **characterized in that** the fluid processing unit further comprises at least one device selected from the group consisting of a filter element (41), a cooling and/or heating device, ventilation equipment, a heat exchanging device (42), a pressure reducer, a pump (40), a pH meter, a conductivity meter, a thermometer, and a manometer.

4. Bioreactor according to any one of claims 1 to 3, **characterized in that** at least one second substrate is provided, said second substrate comprising at least one first surface for carrying phototrophic organisms, wherein the first substrate is disposed such that at least the first surface is exposed to ambient air, and at least one second surface which is at least partially in contact with the fluid path.

5. Bioreactor according to any one of claims 1 to 4, **characterized in that** the fluid path comprises at least one porous material, the porous material preferably comprising a spacer fabric.

6. Bioreactor according to any one of claims 1 to 5, **characterized in that** the first substrate (29) and/or the second substrate comprise(s) a microporous membrane comprising polyethersulfone (PES).

7. Bioreactor according to any one of claims 1 to 6, **characterized in that** the bioreactor (20) further comprises a control unit comprising at least one sensor device.

8. Bioreactor according to claim 7, wherein the sensor device is designed to measure at least one condition selected from the group consisting of temperature, CO₂ concentration, light intensity [µmol/s/m²], filling level, salinity and humidity.

9. Bioreactor according to any one of claims 1 to 8, **characterized in that** a container (26) for collecting the fluid is provided, said container (26) being in fluid communication with the fluid path and the fluid processing unit (28), wherein at least one connecting element (35, 36) comprising a beveled edge (38) is disposed between the fluid path and the container (26).

10. Arrangement comprising the bioreactor according to any one of claims 1 to 9, **characterized in that** said bioreactor is in fluid communication with at least one aquaculture system, wherein waste water derived from the aquaculture system is processed in the bioreactor and then recirculated to the aquaculture system.

11. Method for treating at least one fluid and/or cultivating phototrophic organisms, wherein phototrophic organisms are immobilized on at least one first surface of at least one first substrate, wherein said first surface is exposed to ambient air, and wherein said first substrate is permeable to fluid but impermeable to phototrophic organisms and further comprises at least one second surface which is at least partially in contact with a fluid path which is in fluid communication with at least one fluid processing unit, said method comprising:
a) Processing the fluid by means of the fluid processing unit,
b) providing the fluid by the fluid processing unit to the fluid path,
c) bringing at least a part of the fluid in contact with the second surface of the first substrate, wherein the first surface is provided with the fluid through the second surface,
d) recirculating the remaining fluid from the fluid path to the fluid processing unit, and
e) repeating steps a) to d) at least once.

12. Method according to claim 11, wherein the processing of the fluid comprises at least one sterilization process, preferably at least one UV irradiation and/or at least one ozone treatment of the fluid.

13. Method according to claim 11 or 12, wherein the processing of the fluid comprises at least one treatment selected from the group consisting of filtration, cooling, heating, pH adjustment, addition of water and/or medium, and adjustment of gas content.

14. Method according to any one of claims 11 to 13, wherein the fluid is a liquid solution comprising nutrient compounds for cultivating phototrophic organisms, and/or wherein the fluid is waste water derived from an aquaculture, at least some of the waste being removed from the water by the phototrophic organisms and the cleaned water is then recirculated to the aquaculture.

15. Method according to any one of claims 11 to 14, wherein the phototrophic organisms are removed from the substrate and then directly fed to an aquaculture, or wherein the phototrophic organisms are removed from the substrate and then used to produce fatty acids, protein, food and/or dietary supplements.
